# EUROPEAN PATENT SPECIFICATION

(11) **EP 1 865 905 B1**
(45) Date of publication and mention of the grant of the patent: **27.02.2013**
(21) Application number: 06723317.1
(22) Date of filing: 09.03.2006
(51) Int. Cl.: A61F 13/494, A61F 13/475

(54) **ARTICLE, SUCH AS DISPOSABLE ABSORBENT ARTICLE, MAINTAINING A CONCAVE CUP-LIKE SHAPE DURING USE**
GEGENSTAND, WIE Z.B. SAUGFÄHIGER EINWEGARTIKEL, DER IM GEBRAUCH EINE KONKAVE TASSENFÖRMIGE FORM BEHÄLT
ARTICLE, TEL QU'UN ARTICLE ABSORBANT JETABLE CONSERVANT UNE FORME DE BANDE CONCAVE LORS DE L'UTILISATION DE L'ARTICLE

(30) Priority: 29.03.2005 WO PCT/IB2005/000845
(43) Date of publication of application: 19.12.2007
(73) Proprietor: Concepts For Success, 53881 Euskirchen Stotzheim (DE)
(72) Inventor: SCHMITZ, Christoph, 53881 Euskirchen Stotzheim (DE)
(74) Representative: Plischke, Manfred
(86) International application number: PCT/EP2006/002166
(87) International publication number: WO 2006/102974

(56) References cited:
- EP-A- 0 067 377
- WO-A-93/22997

## Description

### Field of the invention

The present invention relates to an article comprising a waist or leg hoop such as a comfortable garment or a pad to be worn on the lower torso of a wearer for which a three dimensional cup- or bowl-like shape is maintained during use or wear. In one particular aspect, the invention relates to disposable and even more particular to disposable absorbent articles, which can be produced on automated high speed production lines, such as throw-away underwear, baby diapers or adult incontinence products, but also to durable apparel such as girdles, panties, tights and so on.

### Key prior art

Articles intended to be worn on the lower torso of humans are well known such as in the area of apparel, such as trousers or pants, or in the area of hygienic articles such as menstrual pants or so called training pants, but also in the area of diapers or incontinence articles, and further for disposable pads such as used in the feminine care area.

They have in common, that a part or a region of the article is positioned in the crotch region between the legs of the wearer, whilst at least one other region of the article extends forward or rearwards. As this body region is particularly sensitive but also exhibits a complex shape with wide variations for the individual user, many attempts have been taken to address the fit for articles to be worn in this region, in particular for articles, which are intended to satisfy certain functional criteria, such as absorbency and/or containment of body fluids.

Such articles are typically produced by using web materials, such as textiles as woven cloth, non-wovens, and/ or film materials, or combinations or composites thereof. In particular, disposable articles are often produced on high speed production lines for combining such flat webs to form the article. Conventional production lines are much more easily to construct and to operate, if the articles are also flat or can be folded into a flat structure. This is of course often conflicting with the fit requirements of such articles.

Henceforth, there have been many attempts to create designs, which are comfortably and functionally shaped, but yet easy to produce.

In US-B-5615691, a shielding device for the perineal area is described in the form of an elongated pad providing a non-contact zone between the perineal area of the wearer and clothing.

Sanitary napkins or pads which an to be used in connection with menstrual and/ or underwear pants are well known to comprise so called wings, which are typically designed so as to be folded downwardly around the longitudinal perimeter of the leg cut out of the pants. Alternative designs comprise curved hinge lines, e.g. as shown in US-B-4655759 or EP-A-1332742. WO 93/22997 shows absorbent articles, such as sanitary pads, in the form of cup-like shaped structures. In US-A-2005/0107762, chafing barriers are described, which extend from the crotch regions towards the inner thigh region, and which may be applied to conventionally designed pads or hygienic pants.

Further approaches aim at improving the fit of pants or pant-like structures. Well known are essentially two-dimensional articles with elastic members. Simple approaches just use elastic belt like structures in the waist region, other add leg elastics, and even further approaches implement elastic panels in various regions, in particular by using elastic side panels in the hip regions. Other approaches use - relatively expansive - circular knitting technology, such as described in US-B-5590548.

US-A-200510120466 describes an attempt to improve the fit of boxer short type pants by gathering web material around the leg cut out by attaching an elastic strip in the crotch region. A further attempt to arrive at more body-conforming fit is disclosed in US-B-6666851, showing an essentially two-dimensional (2D) disposable garment in pant form, wherein the fit is improved by folding and tucking in of the crotch region.

Yet another type of article for which fit is of particular importance is a diaper, be this for babies or for adults. Typically, such products comprise an absorbent centre piece positioned in the crotch region of the wearer and extending towards the front and/or back, combined with sealing means and closure means for affixing the article around the waist of the wearer, In JP08252281A2 a disposable nappy with portions that seal around the circumference of thighs to prevent leakage is described. Another approach to a diaper design is shown in US-B-06926702 in the form of a diaper having side flaps extending along the legs of the baby, and in US-B-4990147 and in WO-A-04/043317 diapers are disclosed comprising a secondary topsheet or topsheet-like cuffs to separate faeces from skin. Further, there are known many approaches combining elements as described so far, such as disposable diapers for the use as training pants, which are designed in a closed pant form, but which may be openable, and optionally also recloseable, so as to allow inspection by the caretaker, or use of the toilet, e.g. of a toilet training toddler. In US-B-6652497 a disposable absorbent article having a garment like appearance is disclosed and in US-B-4797955 a simple garment as a combination of a skirt and a pant is shown.

In US5827260 and EP0901780 absorbent articles are described comprising elements for encircling the legs of a wearer which are non-unitary with the main body of the article. These elements are connected to the main body such that the inwardly facing surface of the main body is facing the outwardly facing surfaces of the leg elements. In order to adapt to the body contours of a wearer the leg elements are elastized.

However, none of the discussed approaches provide a fully satisfactory approach towards improved fit in the crotch region and around the upper thigh regions, or at least the upper inner thigh region, be it adapted for female, male, or child anatomy and uses, in particular not for articles, which are intended to be produced at low cost at high efficiency automated production lines.

Moreover, none of the articles designed for keeping solid waste materials away from the akin of the wearer by provision of a void space between a secondary body side liner and an absorbent core provides functional means to keep this void space under normal in use conditions mostly open. Henceforth, it is an object of the present invention to provide an article which satisfies the functional a aesthetic requirements, such as fit, containment, discreteness, appearance, and yet can be readily produced at low cost.

### Summary of the Invention

This is achieved by the present invention, which is an article comprising or being adapted to comprise during use a cup-like shaped structure, which is adapted for being concavely positioned in the crotch of a wearer during use. The article comprises a front and/or a rear region, and a crotch region. The crotch region comprises a centre crotch region, and two side crotch regions positioned laterally outwardly of the centre crotch region. The cup like structure is formed and maintained during use by connecting the side crotch regions along connecting lines to respectively adjacent front or rear regions, and/or - in the case, that the side crotch regions are essentially non-unitary with the centre crotch region - to the centre crotch region, whereby at least one of the connecting lines or optional fold-lines is curve-linearly shaped. The cup-like shape of the article may be formed by upwardly bending the laterally outward or inward perimeter of the side crotch regions. The side crotch regions form a hoop, and a waist hoop may be connected to or formed by the front and rear regions. The article may further comprise side panel materials connected to the front and rear regions and to the side crotch regions, whereby the side panel materials are non-unitary materials.

The article according to the present invention may be a disposable absorbent article, a disposable non-absorbent article, a baby diaper, a feminine hygiene product, an adult incontinence product, a piece of single or limited use underwear, a piece of operation theatre garment, or a tight fitting elasticized garment The article may comprise web materials, such as non-wovens, films, or composites thereof. In the various regions, the article may comprise at least partially elasticized materials. The materials or the regions of the article preferably exhibit a cantilever stiffness of less than 20, preferably less than 10, or more than 1.5, preferably 2, when tested according to ASTM-D-1388, optionally exhibiting directionally non-uniform stiffness.

The connecting of the regions may be achieved by connecting lines, which may be continuous or intermittent, curve-linear or straight, single or multiple lines, intercepting lines, or defined as the perimeter of a connecting region. The connecting may be permanent, preferably is a non-sewn connection, optionally with tear open aids, or refastenable system of the hook-loop type, or a refastenable or permanent adhesive system.

The article may further comprise any or all of an absorbent core, a barrier leg cuff a secondary topsheet, or lotion application to body facing surfaces.

### Brief description of the figures

- Fig. 1 A-C: show schematically a conventional cup-like shaped article in perspective, top, and cross-sectional view;
- Fig. 1 D-E: show schematically a conventional cup-like shaped article in a perspective and in a partially exploded view;
- Fig. 2 A-C: show schematically designs for cup-like shaped articles in their pre-use flat configuration;
- Fig. 3 and 4: show schematically various embodiments for 3D-shaped pants;
- Fig. 5 A-C: schematically shows closed or hoop structures;
- Fig. 6 A-B: depict process schemes for the manufacturing of articles according to the present invention;
- Fig. 7 A-C: show schematically fit lines in the crotch region;
- Fig. 8: depicts schematically a conventionally shaped absorbent pad,
- Fig. 9 A-D: schematically shows force and fit lines of conventional diaper designs;
- Fig. 10: schematically depicts force lines for a pant-like article according to the present invention;
- Fig. 11 A-B: shows schematic fit lines for conventional diaper designs comprising a secondary topsheet
- Fig. 11C: shows schematic fit lines for a pant-style diaper with a secondary topsheet according to the present invention;
- Fig. 12 A, B: show a schematic perspective view of a pant-style diaper with a secondary topsheet according to the present invention;
- Fig. 13 A-B: depicts an alternative execution of a pant-style diaper with a secondary topsheet according to the present invention

The same numerals in various figures refer to corresponding elements.

### Detailed description

An article in the sense of the present invention is an article such as a comfortable garment or a pad to be worn on the lower torso of a wearer for which a three dimensional (3D) cup- or bowl-like shape is maintained during use or wear and which comprises a waist or leg hoop. In particular aspects, the invention relates to disposable and even more particular to disposable absorbent articles, which can be produced on automated high speed production lines, such as throw-away underwear, baby diapers, or feminine hygiene or adult incontinence products, but also to durable apparel such as girdles, panties, tights, and so on.

Directions and orientations for such articles are herein defined in the context of a wearer when the article is worn, typically in an upright standing position. In this context the terms lower, upper, outer, inner, front, rear, left, right, or lateral are used in the anatomical definition.

Thus, within the context of the present invention, the garment has a lateral direction (y direction, cross-direction (CD), or width) defined as the direction parallel to the lateral centreline and being aligned with a "left-to-right" direction of the wearer when being used; the longitudinal direction (x direction, machine direction (MD) or length) being defined as the direction parallel to the longitudinal centreline and being aligned with the height direction of a wearer in a standing position during use; and the thickness or z-direction essentially perpendicular to the other.

When an article or a piece of material extends during use from the back of a wearer through the crotch region to the front waist region, the MD-line may be U-shaped. The CD-line may be left to right, and the thickness orientation will generally be perpendicular to these, such that it would point "forward/rearward" in the back and the front regions of the "U", and "upwards" in the centre region of the U or the crotch region of the wearer.

Within the present context, an article comprises several regions, especially a crotch region, which is intended to be positioned in the crotch region between the legs of a wearer during use, and a front and/or rear region, extending forwardly respectively rearwardly and upwardly during use. Any region may comprise sub-regions, such as the side crotch regions laterally adjacently positioned to a centre crotch region.

A region on an article refers to the whole of the article in this region when being in an in-use configuration, and thus may comprise various materials, often in a layered arrangement. However, during the manufacturing or even after manufacturing, materials of two different in-use regions may lay on each other, for example when an article having a three-dimensional shape during use is folded, optionally flat, in the packaging.

Within the present context, a line extends between at least two non-identical points. A line may be a continuous line, or it may comprise a multitude of line segments or dots arranged along a geometrical path. A physical line in the present context (e.g. a glue bonding line as connection line) will have a certain width, and may even be seen as an area or region. Such a region is circumscribed by its perimeter line, which may have a characteristic forum, such as being straight or curve-linear, and the region can then be described as straight perimeter region or a curve-linear perimeter region.

An article according to the present invention has at least in its in-use configuration a cup- or bowl-like three-dimensional configuration, whereby at least parts of the article positioned in the crotch region during use define an "upwardly open" or concave structure.

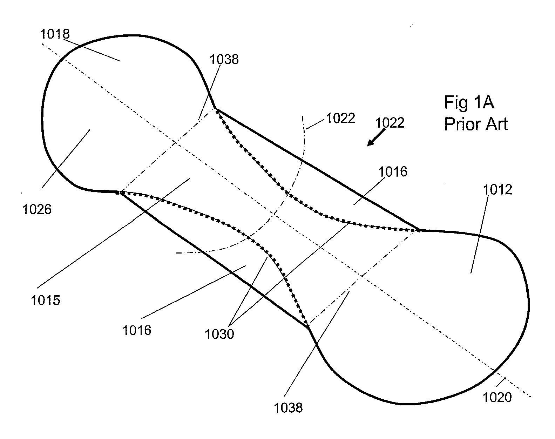

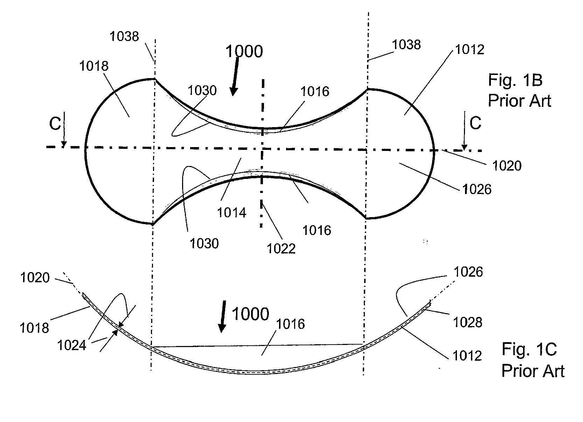

This is further explained in Fig. 1A showing the perspective view, Fig. 1B showing a top view, and Fig. 1C a cross-sectional side view of a simple conventional cup like structure 1000, having an x-direction along a longitudinal centreline 1020, and a width direction along a cross-directional centreline 1022. Such an article comprises a crotch region 1015 and a front (1012) and a rear (1018) region separated from the crotch region by a separation line 1038. The crotch region is essentially a unitary and continuous region (i.e. not composed by connected pieces), comprising sub-regions, namely a centre crotch region 1014 and laterally adjacent side crotch regions 1016, which are delimited from the centre crotch region by a curve-linear fold line 1030, which may have been created e.g. by embossing, or by other appropriate means, such as profiled lay down of absorbent material positioned under the fold lines. Such an article will take a cup-like shape upon upwardly folding the side crotch regions 1016 along the fold-line 1030. It will, however, only maintain this shape, e.g. during use, if there are forces to hold it in this shape, e.g. when it is positioned in a supporting panty. Such cup-shaped articles have an "inner" (1026) and "outer" (1028) surface spaced apart by the thickness of the article along the z-direction 1024. When the article is worn, the inner side is oriented towards the wearer, and the outer surface is opposed. However, during production such an article may be "inverted", e.g. it may be produced "inside out" and then be turned or inverted.

Similarly, such articles can be described by a space, which is "inside" of the article, and the complementary space, which is outside of the article. The "inside space" is thus enclosed by the inner surfaces and - if these do not form a closed space - by one or more hypothetical cover(s) closing the space. For articles of more complex shape, inside refers to the smallest space enclosed by the inner surface of the article and the smallest cover area required to close the space. Hence, for a pair of trousers, the inside space is formed by the inner surface of the article and the areas of the waist opening and, the two leg openings. For a simple cup-shaped or concave article, the inner space will correspond to the volume of the bowl, whereby the surfaces will be complemented (if required) by the smallest cover areas.

The present invention relates to articles or garments to be worn or applied in the lower torso region of the wearer, which extend at least partially into the crotch region of the wearer.

A particular application relates to pants and trousers, which typically encircle the waist or hips of the wearer as well as the legs at least in the upper thigh region of the wearer. Trousers or pants may comprise integral or separate means for being held on the wearer, such as belts, suspenders or braces, each of which may comprise or be made of elastic materials. Such pants may be used as underwear, girdles etc, but may also be worn on the outside, such as sportswear, swimwear, etc. Pants may be designed to be re-useable or disposable; the latter may be applicable to underwear or applications in the operation theatre, therein combined with sterile properties. Such pants may comprise absorbent elements or materials. Pants may have stockings connected thereto, such as in the form of a pantyhose or tights, which are close fitting coverings of the body from the waist to the feet, most frequently worn by women.

A further application for articles being worn in the crotch region are pads, shields, or dressings, which are often designed to be absorbent and disposable. In contrast to pants, these do typically not encircle the waist of the wearer, but comprise leg hoops, and which may additionally be hold in place on the body by support means, such as garments like a underwear, sportswear, or menstrual pants. In addition or alternatively, adhesive means may be used to attach the pads to such garments or directly to the skin of the wearer.

Yet a further application area for articles being worn in the crotch region are diapers. Diapers typically comprise absorbent elements at least in the crotch region and are designed to be disposable, i.e. they are intended to be discarded after being loaded with bodily exudates like urine or faeces.

In addition, the present invention may be related to combinations of features as described hereinabove, such as pant-shaped diapers, which are permanently closed to form a pair of pants, and which may comprise a "tear open" feature, allowing the care taker to more easily remove the diaper from a baby. Or the diaper may be pre-closed by the manufacturer to form an openable pant-like structure, such as by recloseable features, or also by macro fastening devices comprising buttons and button holes, or by slot-and-tab fastening means.

Similarly, there may be pad and pant systems wherein either or both of the elements are particularly adjusted to interact with the other, such as when a pad with hoops is particularly formed to fit into a particularly adapted pair of pants. Typically, though not necesserily, the pads are absorbent and disposable whilst the pants may be re-useable and washable.

Such articles may also be swimwear with features to capture bodily exudates, which may be re-useable if not soiled by the wearer, but which may be disposed of, if soiled. It may also combine diapers or other pant like structures with skirt like structures.

As the articles of the present invention may be produced on high speed production lines, they are of particular relevance for disposable absorbent articles of any of the types described hereinabove, allowing low cost production so as to minimize cost for the user. The term "disposable" is used herein to describe absorbent articles or elements which are not intended to be laundered or otherwise restored or reused as an absorbent article or element (i.e. they are intended to be discarded after a single use and, preferably, to be recycled, composted or otherwise disposed of in an environmentally compatible manner).

Disposable absorbent articles as well as various elements comprised therein are well known to the person skilled in the art in multitudes of executions and henceforth a number of generally known details are omitted herein. They are typically worn to receive bodily exudates, such as urine, faeces, menses, sweat, etc. and thus may comprise liquid absorbing materials, such as - without any limitation - cellulosic fluff and/or superabsorbent materials. Absorbent articles typically comprise further elements to retain the bodily exudates within the article, respectively within the space between the article and the body of the wearer whilst avoiding soiling such as of other garments. Typically, such articles comprise sealing elements, such as elastic elements to create a gasketing effect at the perimeter of the article, e.g. legs and waist. The articles typically comprise liquid impervious materials to contain body fluids. Many of the materials used in such articles are web materials, i.e. flat materials supplied in an essentially endless form such as on rolls or spools, such as films, non-wovens, woven materials, tissues and the like. In addition, the articles typically comprise other materials, such as particulate materials, such as so called superabsorbent materials, or fluids, such as adhesives or skin care fluids.

The concave cup-like shape as a first element of an article according to the present invention is achieved and maintained during use by connecting the side crotch regions, such as by bonding, to adjacent regions.

In a first embodiment, side crotch regions are connected to the respectively adjacent front or rear regions of the article.

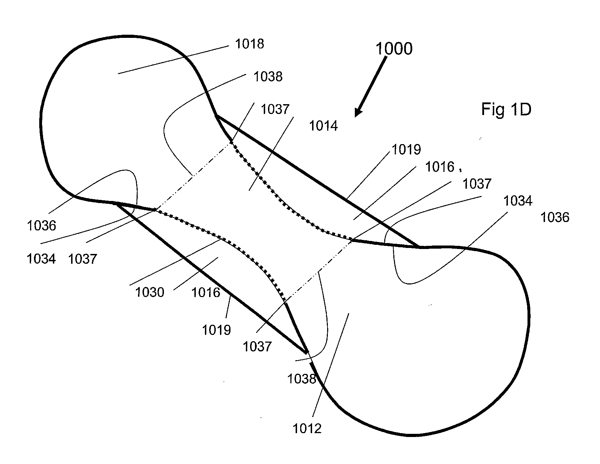

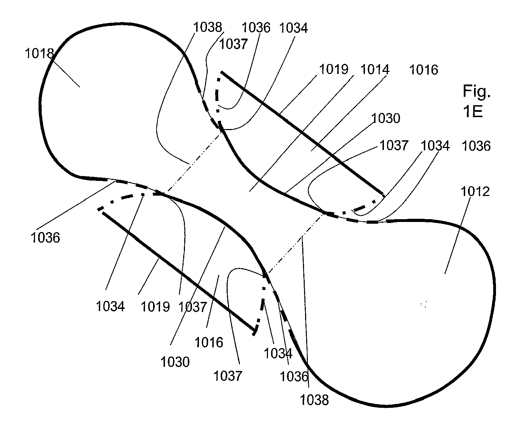

A first exemplary element for this execution is described in Fig. 1 D, showing a perspective view of such a cup-like shaped pad, such as might be a feminine care pad, and Fig. 1E showing a schematic exploded flat top view of the article 1000 with front (1012) and rear (1018) regions and a centre crotch region (1014) connecting these two. The side crotch regions 1016 may be unitary with the centre crotch region 1014 delimited there from by the curve-linear fold line 1030. As can be seen in Fig. 1E, this fold-line 1030 does not extend fully towards the laterally outward side margins 1019 of the side crotch region 1016, but terminates at an intercept point 1037. Looking outwardly from this point onwards, the side crotch region is delimited by a cut line 1034, which extends essentially cross-directionally. When the article is cut open and laid out flat as in Fig. 1E, a semi-crescent cut out region between the side crotch region and the front and rear region appears. This cut-line 1034 is connected to bond line 1036, which extends from the intercept point 1037 essentially fore-respectively rearwardly. This connecting effectively foreshortens the laterally outwardly sections of the pad, and thus creates and maintains the cup-like shape. In this design, the centre crotch region 1015 is delimited from the front (1012) and rear (1018) regions by an essentially CD-oriented separation line 1038 connecting corresponding intercept points 1037.

Preferably, the foreshortening and the relative arrangement of the cut-line to the bond line will create at most a perpendicularly upstanding side crotch region relative to the centre crotch region.

The skilled person will readily realise, that the connecting between the front and rear region and the side crotch regions may also be achieved by straight, instead of curved, cut and bond lines. Thus, the semi-crescent shaped cut-out would look like a triangle or rectangle. Similarly, the cut lines may be replaced with appropriately arranged fold-lines, whereby the material corresponding to the cut-out would be folded away to allow the foreshortening.

It will also be appreciated, that a cup-like shape will already be created if the bonding of the side regions to one of the front or rear regions only, e.g. when the article does not comprise the respectively other regions.

A second element for creating and maintaining the cup-like shape comprises side crotch regions, which are essentially non-unitary with the centre crotch region, such as is achieved by adding a leg hoop to encircle the thigh of the wearer. For this element, at least one of the connecting lines or optional fold-lines is curve-linearly shaped.

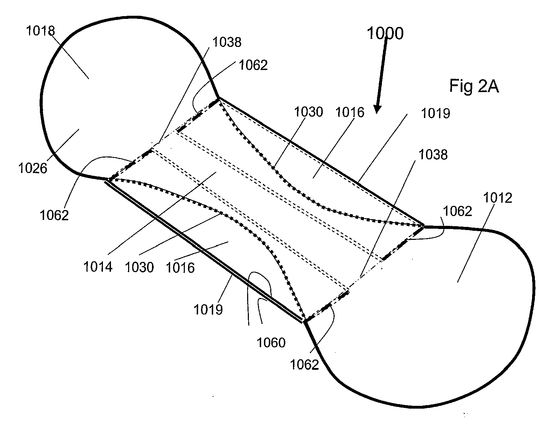

A first exemplary article comprising such an element is depicted in Fig. 2A. Fig. 2A depicts the article 1000 showing the user oriented inner surface 1026 still in a flat state, with front (1012), rear (1018) and centre crotch (1014) regions, whereby the latter is delimited from the first two by lines 1038. The side crotch regions 1016 are each made of a double layer material forming the leg hoops 1060. The laterally outer perimeter of the centre crotch 1014 region falls together with the curve-linear fold or connecting line 1030, which is bonded to the contacting surface of the leg hoop material 1060, such that parts of the leg hoop materials 1060 form the side crotch regions 1016, such that the reminder of the leg hoop materials lays on the outer side of the garment, opposite to the wearer oriented surface 1026. The two layers of the leg hoop material are connected to each other e.g. at their longitudinal perimeter by a hoop bond line 1062 or any other connecting means to form the closed hoop structure. Their width is preferably more than 1 mm, more preferably more than 5 mm, and even more preferably more than 10 mm, but preferably less than 100 mm, more preferably less than 70 mm, and most preferably less than 40 mm. Their shape can be rectangular or of any other shape identified to be best for containment and comfort of the wearer. When applied on the user the leg hoops can be of basically cylindrical shape or any other shape identified to best meet the design intend of the article

Upon use, the lateral outer perimeter 1019 of the side crotch regions 1016 are folded upwardly and opened to form leg openings, thereby - because of the curve-linear shape of the connection line - forming the centre crotch region into the cup-like shape. Optionally, and often preferably, the lateral outer perimeter 1019 may also be curve-linearly shaped.

Due to the fixation around the legs of the wearer, this embodiment stays appropriately positioned on the body without any further support means; although it may be used in connection with other support means, such as pants, or body adhesives.

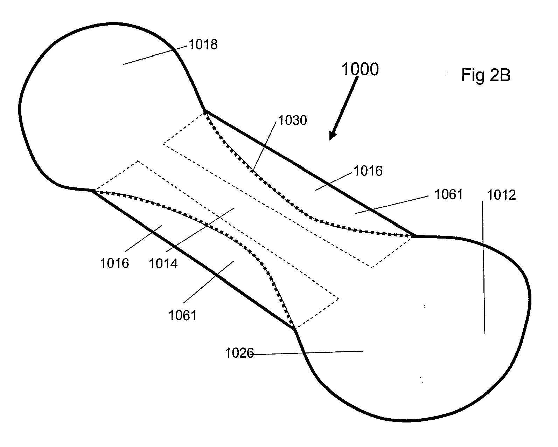

An alternative article design not encompossed by the present invention is depicted in Fig. 2B, differing in that instead of a dual layer hoop material, a single layer material is used as a leg seal cuff 1061, which is intended to lie against the inner thigh region of the wearer, and thus - in addition to creating the cup-like shape - aids in preventing soiling. Provided the leg seal cuff materials exhibits a certain stiffness against cross-directional folding, also this design maintains its cup-like shape without the need for maintaining forces or means.

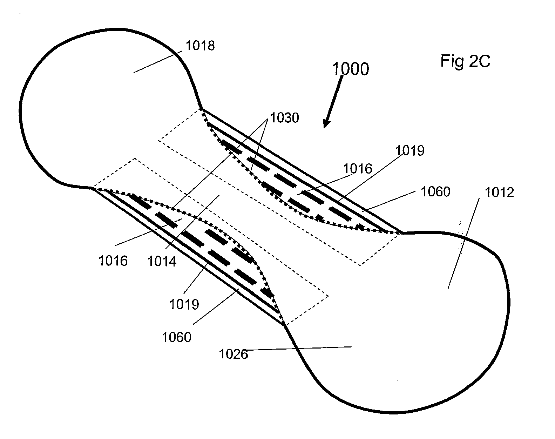

Whilst these examples showed on each side a curve-linear connecting line 1030 between the centre crotch and the side crotch regions, instead of this one line several lines may be used, such as essentially parallel lines, or an interrupted line pattern such as a dashed-dotted-line) may be used. The connecting may also be achieved by a connecting area. In any of these alternatives, the circumscribing perimeter of the connecting region can be described by a curve-linear line. In Fig. 2C, the laterally outward side margins 1019 of the centre crotch region 1014 is positioned more outwardly than the fold line 1030, which may also be a delimiting core perimeter line of a bonding area between line 1030 and the side margins 1019. Thus, the connecting line may be a straight or any other shape of connecting line, if this is accompanied by a curve-linear folding line. In the above examples, the connecting line has actually a dual function, namely the connecting of the regions, and the forming and maintenance of the cup-like shape, e.g. when being unfolded into the in-use configuration. Such as depicted in Fig. 2C, these two functions can be separated, and in this case, the connecting line may be e.g. a straight line or several straight lines or a bonding region having a straight line perimeter.

The above described two embodiments are not exclusive, but may complement each other. Thus, an article may have the side crotch regions connected to the front / rear regions, and it may have a curve-linear connecting or fold line between the side and centre crotch regions.

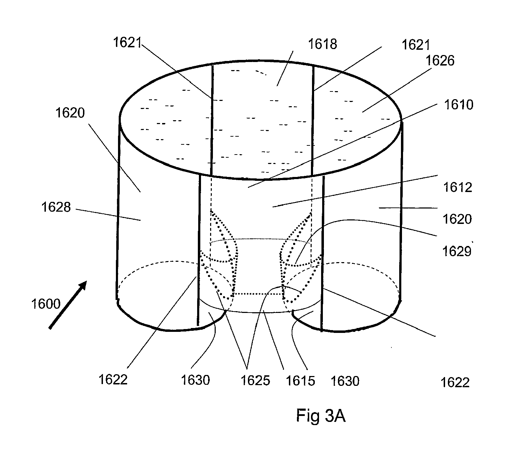

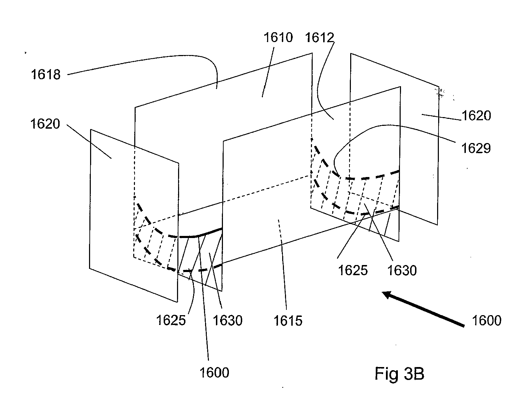

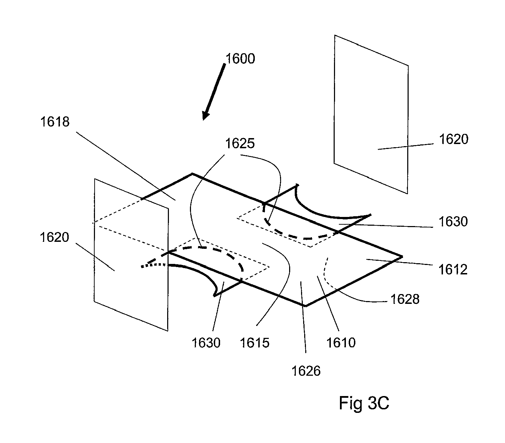

An exemplary execution for such an article is described together with Fig. 3A showing a perspective view and Fig. 3B showing a schematically partly exploded view of a pair of pants with a leg seal cuff. A centre piece 1610 of the article 1600 extends from the rear waist region 1618 through the crotch region 1615 to the front region 1612. In the waist region of the article, side panels 1620 are connected to the centre region by front (1622) and rear (1621) connection lines. In the crotch region extending towards the thigh region of a wearer during use, additional leg seal cuffs 1630, forming the non-unitary side crotch region 1016 are connected along the downward continuation of these lines to the side pieces 1620. They are also connected to the centre piece along connecting lines 1625, preferably executed in a curved shape, thereby supporting the cup-like shaped structure of the article. Also the upper margin of the leg seal cuff 1629 is preferably executed in a curved shape. The width of the leg seal cuffs is preferably more than 1 mm, more preferably more than 5 mm, and even more preferably more than 10 mm, but preferably less than 100 mm, more preferably less than 70 mm, and most preferably less than 40 mm. Their shape can be rectangular or of any other shape identified to be best for containment and comfort of the wearer. Fig. 3C shows a partly exploded view with the centre piece being cut out and laid flat. The leg seal cuffs 1630 are attached to the outer surface 1028 of the article.

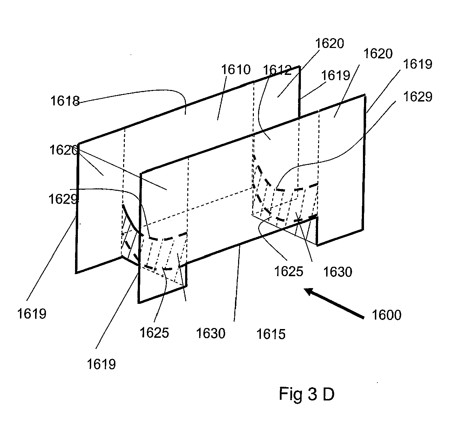

The side panels 1620 may also be designed as unitary or pre-connected lateral extensions of the waist front region 1610, as depicted in Fig. 3D. When these side panels are connected at their outer perimeter 1619 they form the closed pant structure, encircling the waist with front (1612), rear (1618) regions and the upper parts of the side panel extensions 1620, and the legs with leg seal cuffs 1630 and the lower parts of the side panels 1630.

Such a hoop or leg seal cuff design provides particular benefits for articles, which require "acquisition space" (diapers), tight but gentle sealing in the leg region (Feminine hygiene and especially menstrual pants), or gentle distribution of forces (girdles), or provide a sustained dynamic fit for the wearer. In addition to the gentle distribution of sealing forces, it is believed, that a particular benefit may come from the connecting of the leg seal elements to the centre crotch region. Apart from creating the cup-like shape, and thusly creating the particular benefit of a void or a non-contact zone between the article and the skin of the wearer, the force distribution along the leg seal cuff or hoop is not along the outer (or in the in-use positioning along the upper or lower) perimeter of the sealing element, but more towards the centre region thereof, thusly allowing functional separation of the sealing and the support functionality.

There are various specific embodiments of this design principle, such as having certain regions of the article elasticized, e.g., the leg seal cuff may be elastically extensible in front to back direction only. Also, the side panels may be elasticized, optionally with a varying degree and / or directionality of elastication in different regions. The side panels may be openable, such as by being slit in combination with a re-usable closure system, such as mechanical fastener. The side panels may also be composed of two connected sub-panels, e.g., made of a first material in the upper part (i.e. reaching into the waist region), and a second one made of a second material in the lower part.

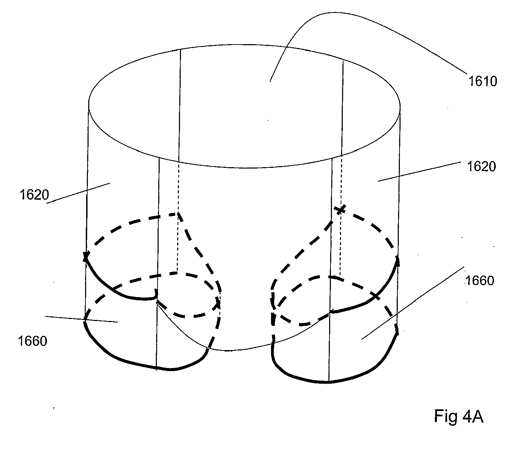

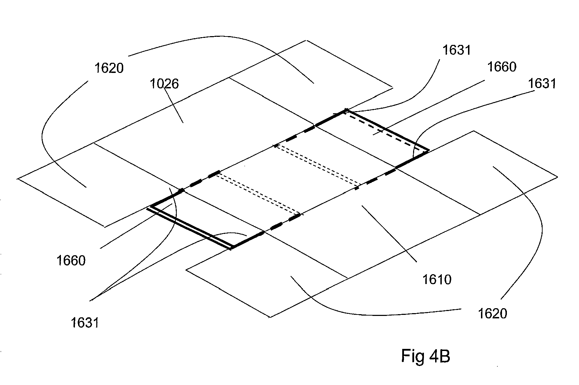

A further exemplary execution is depicted in Fig. 4, showing pant shaped article comprising a leg seal cuff extending from the inner thigh region all around the leg, thereby forming a hoop (see Fig. 4A showing perspective view, and 4B, showing the article partly cut open and laid flat). The leg hoops are here produced out of two layers of material, which are connected by bond lines 1631. Thus, such a design would also allow the outer hip portion of the pant to be composed of two different materials-one extending to the waist region and the waist opening, and the other one extending to the leg opening. The width of the hoops is preferably more than 1 mm, more preferably more than 5 mm, and even more preferably more than 10 mm, but preferably mess than 100 mm, more preferably less than 70 mm, and most preferably less than 40 mm. Their shape can be rectangular or of any other shape identified to be best for containment and comfort of the wearer. When applied on the user the leg hoops can be of basically cylindrical shape or any other shape identified to best meet the design intend of the article

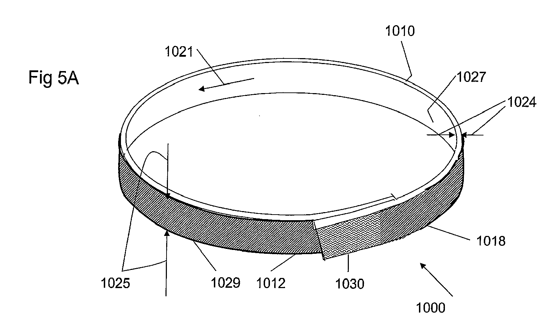

In the present context, a hoop refers to a closed structure such as a circular band of material. Typically, though not necessarily, it comprises flexible and often web materials, such that the form is not necessarily always circular, but it may be of elliptical, oval or irregular shape, e.g. by following the shape of a leg or a waist when encircling these. A typical embodiment of a hoop is a belt encircling the waist. Such a hoop, as depicted in Fig 5A may have a x-or longitudinal axis which would correspond to the waist encircling line, a thickness dimension 1024 perpendicular thereto, and a width dimension 1025.

Essentially a hoop consists of an outwardly (1028) and an inwardly (i.e. towards the body - 1026) facing surface, positioned at a distance corresponding to the hoop thickness 1024 (or web material thickness), and upper and lower opening. For hoops with relatively small width, the upper and lower openings are not necessarily distinguished, such that a belt like hoop may be considered to form the waist opening or the leg opening.

A hoop being useful in the present invention may be made of flexible web material so as to conform to a specific (human) body part. It may be extensible or elastic, such as by being shirred in combination with an elastic member, or it may be knitted, e.g. by tubular knitting.

A hoop may be a composite of several materials, be this in a way of e.g. homogeneously mixing material like fibres in a web material, or by creating a layered structure by assembling two or more materials in the z-direction of the hoop, or by placing different materials longitudinally adjacent to each other in the y- or width direction of the hoop, or by connecting one or more pieces circumferentially along the x-direction of the hoop.

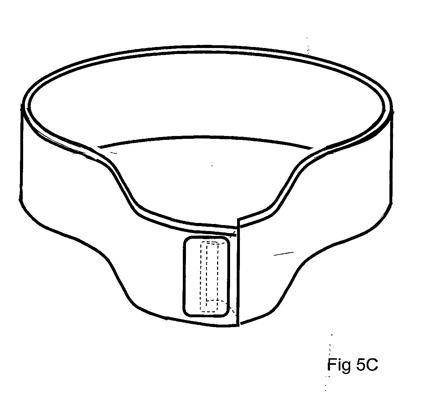

A hoop may have a constant width 1025 or the width may vary. It may have a cylindrical shape, or it may be irregularly shaped, as depicted in fig 5C. This may be done to improve fit on the wearer. It may also be done to ease gripping such as by shaping a grip flap for example to ease donning by the wearer or a caretaker.

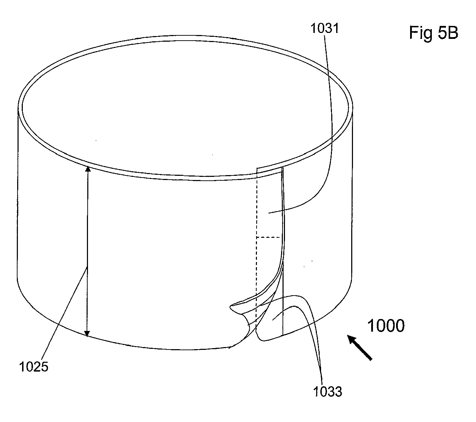

A hoop may be permanently or temporarily closed, such as by connecting two ends of a belt by any of the connection elements described hereinafter. In particular embodiments a hoop may be partly openable, such as depicted in Fig. 5B, where the connecting region is executed partly as a permanent bond (1031) and partly refastenable (1033), such as by hook-loop fastening system, which can be used to adjust the hoop diameter.

Typically, though not necessarily, a hoop may be formed by essentially flat materials like a band, ribbon, string, or a panel, or a piece or length of material. A hoop may be a unitary piece of material, such as shown in Fig. 5 A-C.

In the context of the present invention, the term unitary refers to a piece of material essentially forming one or more regions, the material piece or article along the extension of a MD/CD-plane. Thus, for example, if a diaper comprises a unitary backsheet covering the entire article, other materials, which are directly or indirectly connected thereto, - such as topsheets, the absorbent core, etc. - may form the total article. In contrast, if the outer surface of the article is comprised of non-elastic film combined with elasticised side panels connected thereto, this will not be a unitary design. As already shown in Fig. 2A, a hoop may also be comprised of two pieces of material, e.g. when laying two bands 1060 flat on each other and connecting the longitudinal end margins 1062.

When a hoop material or a leg seal material is executed as elastic or elasticized material, it may be preferred to execute the adjacent parts in the centre crotch region also at least partly elastic or extensible. Application of hoop material in elongated or pre-tensioned state, and subsequent relaxation of the product after combining until use is one of several options to benefit from the elastic features of the hoop material.

The cup-like shape of the article according to the present invention is created and maintained by connecting the side crotch regions to other regions of the article, such as are rear or front regions, leg cuff regions and hoop structures or other regions or sub-regions.. Connecting refers to any way of at least temporarily holding the regions together, and hence terms like joining, combining etc. may be used interchangingly.

A typical connection is a bond line, such as an adhesive line, or melt-fusion bonding. The line may be continuous or intermittent (e.g. bonding dots positioned along a line). Such a bond line may be a straight line, as indicated in Fig. 2C, or curve-linear. There might be a single bond line, or a plurality thereof. Also, bond lines may intercept, e.g. forming a X-like pattern. The connection may also be over a connecting area, such as when spray glue is or a multitude of bond lines are applied. In this case, the characteristic property of being curve-linear or straight then refers to the perimeter of the connection. As described in the above, a characteristic description of the connection area is the perimeter as being straight or curve-linear. In case of applying adhesives, these may be applied in an essentially non-sticky or at least low-tack state, which may be activated at a later stage in the process, e.g. by heat, radiation, or pressure.

Any connecting may be essentially permanent, i.e. it can only be destructively opened. This opening may be assisted by specifically designed tear lines, e.g. to allow more controlled opening when removing a soiled pant type diaper.

The connecting may also be openable and reclosable, such as when refastenable adhesive tapes are used, or "hook / loop "type closures (e.g. "Velcro ®"). Also closure systems employing a button and hole system may be employed or macro-fasteners, such as of the slot and tab type. Also conventional belt buckle systems represent a recloseable connection system (see Fig 5C).

A particularly beneficial execution of the connecting relates to connecting elastic, elasticized or extensible materials, e.g. when an elastic leg hoop is connected to an extensible centre crotch region. It may then be preferred to design also the connection as an extensible so, so as to not hinder the stretching of certain parts. This can be achieved by using appropriate materials for e.g. for core making and core wrapping, as are known to persons skilled in the art.

The articles may comprise various other functional or aesthetic elements, which do not require a fully detailed description, because they are well known to a person skilled in the art.

If the article is a disposable absorbent article, an absorbent core may be positioned in the centre crotch region, extending more or less into the front and/or rear regions. Optionally, further absorbency may be designed into side crotch regions, or into the leg seal cuffs. The amount of absorbency as well as other absorbency properties such as acquisition, distribution and retention properties may be adjusted to the particular use. For example, for panty liners only very low absorbent capacity may be required, or leg seal cuffs may be furnished with a small amount of absorbent capacity, e.g. to maintain dry skin. In contrast, such as in particular for adult incontinence applications, the absorbent capacity may be significantly higher and also be distributed over several or all regions of the article. The absorbent cores may be of any conventional type, a preferred embodiment comprises superabsorbent material. For ease of processing, it may be preferable to use prefabricated ("roll stock") cores, which may just be cut to the appropriate size and fitted into the article or other elements making up the article cup shaped structure. In addition to liquid retention materials, the core may comprise other liquid handling elements, such as liquid acquisition members, such as open structure webs, or liquid distribution members, such as wicking materials.

Any of the regions of the article may be elasticized or at least expandable. This refers to the fact that the materials in these regions may extend upon pulling, or retract upon loosening of the pull forces. Preferably, such materials will retain their original shape after the tensioning forces are released. However, also a certain degree of permanent extension may be tolerable or even desired to improve fit.

In particular, the hoop structures may be elasticised, by elasticising any or all of the elements thereof. For example, a waist encircling hoop structure formed by side panels and front and rear regions of the centre piece may be elastically extendible to adjust to varying waist sized by elasticising either the side panels, the front region, or the rear region, or all, optionally to a varying degree of elastification, respectively differing contractive forces at a given extension.

The elastification may also vary across the width of a hoop, such as when in the just described waist hoop the region close to the upper waist perimeter has higher elastic contraction forces than the lower regions, e.g. the ones encircling the hips.

Whilst the present invention provides very good sealing and containment functionality, additional sealing elements may be included, such as so called "barrier leg cuffs" or other leg or waist encircling cuffs. Also so called secondary topsheets may be very advantageously used in the present invention, as explained in more detail herein below.

The article may further comprise aesthetical elements, such as to improve visual appearance such as by colouring or printing, or olfactory elements such as perfume or odour absorbents.

It may also comprise cosmetic or medicinal components, such as skin care compositions applied to skin contacting regions. This may be particularly beneficial for leg seal cuffs or for the above described secondary topsheet. The article may also comprise micro climate regulating components, such as convective air flow enhancements, or temperature regulating materials, as known in the art. Generally, the article may be produced from a wide variety of materials, preferably at least one being a web material.

Generally, the term "web" relates to any material, which is essentially endless or continuous in one direction (generally denoted as "x-direction" or "machine direction"). Webs are often, but not necessarily, stored, supplied, or used in roll form and thusly also sometimes denoted "roll goods". Whilst these are then not "endless" in the strict sense of the word, their extension in this x-direction is significantly larger than in any other direction. By combining consecutive rolls or other batches, ("splicing") such webs can be considered "endless" for all practical purposes. Webs may be transported in a "batch" form, such as when a roll thereof is shipped, or they may follow a "web path", such as when the webs are unwound from a roll. Within the context of the present invention, also the cut pieces of a web material are considered to be a web material.

Thus, the web materials may be textile webs, such a (flat) knitted materials or woven materials. Often, the web materials are selected from the class of non-woven, or film materials, or composites thereof. Any of the materials may be "breathable", i.e. allowing air but not liquids to penetrate through. The centre piece may include absorbent material or it may be combined with an absorbent core. The leg seal cuffs may have a certain liquid or moisture absorbent capacity, which may be designed to capture liquid leaking from the centre piece absorbent structure, or which may be designed to absorb sweat.

The materials useful for the present invention must primarily satisfy the in-use requirements, in particular also a certain strength, stiffness or softness, bendability, smoothness, if required absorbency, etc.... If these properties would be outside such normal use ranges, they might also negatively affect the functionality of the present invention. Thus, a certain stiffness should not be exceeded to allow shaping of the cup-like shape, whilst a certain minimum stiffness will enhance the maintenance of this shape. Stiffness may be described by various parameter, a suitable method has been found to be ASTM D 1388, referring to the cantilever stiffness test. The test is accomplished by placing a one-inch wide (2.54 cm) strip of the web on a horizontal surface, one end of which abuts against the top end of a 41.5° inclined plane. The test sample is placed with its narrow edge at the juncture of the horizontal and the inclined surface. It is then moved forward over the edge between the two surfaces until the free end bends over and contacts the inclined surface. One half of the length of the fabric between the point of departure from the horizontal surface and the point of contact with the inclined surface is the cantilever stiffness length. Preferably, materials for being used in the present invention, the cantilever stiffness values are at least 1.5, preferably more than 2, but not more than 20, more preferably not more than 10. Stiffness values of a material, and in particular of composite materials, may differ in various directions, e.g. be higher in cross-direction of the material, and lower in the machine direction.

In addition the materials may need to satisfy certain processing requirements, yet again strength, stiffness, but also allow connecting, such as by being adhesively or thermally or ultrasonically bondable.

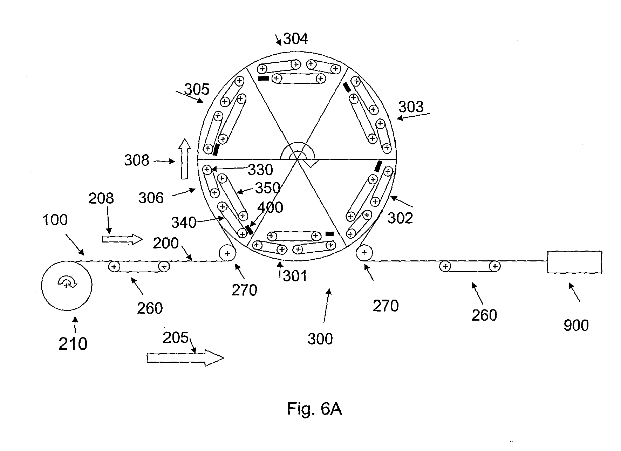

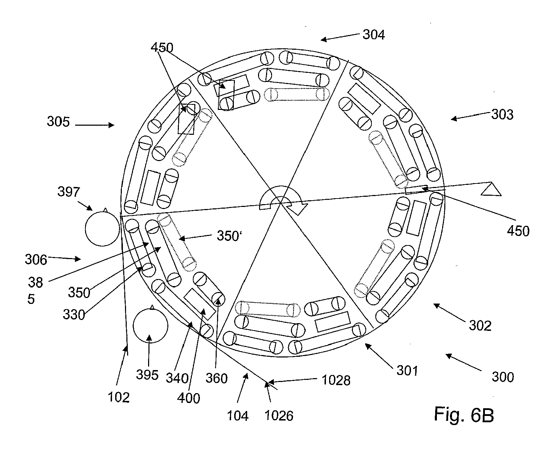

Whilst the present invention allows the application of relatively low speed connecting processes, such as sewing, a particular benefit is that it also allows the manufacturing of the complexly shaped articles with simple, cheap and fast processes, such as thermal or adhesive bonding. In this respect, the present invention not only allows to use mass production materials such as non-woven materials, but also allows production on robust high speed manufacturing equipment, such as schematically depicted in Fig. 6 and explained in further detail in co-pending patent application WO2006/103487.

This method comprises the use of web materials, which are essentially flat by having a length dimension (x- direction) and a width dimension (y- direction) exceeding the thickness dimension (z-direction), thus having a first and a second surface along the x-y-direction. The web materials may form a sequence of web material pieces. The method is an essentially continuous manufacturing process, comprising at least one web treatment step acting on the inner and outer surfaces simultaneously by a web treatment unit comprising a web treatment head and a counteracting web treatment tool. The manufacturing equipment comprises a web path splitter means for parallel treatment of at least two web pieces in at least two web treatment sections, each of these sections comprising a treatment head of the web treatment unit and at least a first and a second web support means for temporarily holding and moving the web material pieces. Thereby, the second web support means is positioned z-directionally offset relative to the first web support means, thus forming a gap between the first and second web support means, wherein the gap distance between the first and the second web support means can be varied.

Fig. 6 shows schematically an exemplary general process set-up, wherein the web path splitting means is a rotating drum (or turret) 300. A web material 100 is unwound from a web supply means 210 along a web path 200, at an overall web path speed 208. The web 100 is transported along the overall process direction 205 by web transport (260) and web guide (270) means via the web path splitting means 300 towards a process end section 900, where it may be transferred to other process units, or e.g. to a packaging station.

In the shown embodiment, the web path splitting means is a rotating wheel 300, comprising six essentially identical web treatment sections (301, 302, ...306). It rotates at a speed, such that the tangential speed 308 at the outer surface of the turret corresponds to the overall web path speed 208. The treatment sections comprise at least a first (330) and a second (350) web treatment means, and a web treatment head 400. A further web support means 340 may be employed, and - as shown in Fig. 6B - an even further web support means 360 may be used. Fig. 6B further depicts the variant of two webs (102 and 104) being fed to the turret 300, each of which may be cut by a web cutting means 397 and 395, respectively. The web support means 330 and 350 are affixed in a moveable arrangement, such that the distance between these two may be varied (indicated in Fig. 6B by the dashed position 350'), and the positioning of the treatment heads 400 can also be changed, such that it can be moved between the web support means 330 and 350. The web treatment heads interact with one or more web treatment tools 450, such as to create a bond between two webs affixed to the treatment head, and treated by the treatment head on one side and the treatment tool simultaneously on the other side.

The method comprises steps of
(a) feeding one or more pieces of web material 100 via the web path splitter means 300 to the web treatment sections 301, 302, ... 306;
(b) temporarily attaching a first region of a piece of a web material 1012 to the first web support means 330 and a second region 1018 of the same or a different piece of a web material to the second web support means 350, such that the first and the second regions are positioned in the gap between the first and the second web support means;
(c) increasing the z-directional distance of the first (330) and second (350) web support means whilst the first and second regions of the web material(s) remain at least partly affixed to the respective web support means, so as to increase the gap distance between the web support means and the regions of the web material piece(s) affixed thereto,
(d) positioning the web treatment head 400 in the gap to contact the surfaces of the web material regions oriented towards the web treatment head;
(e) positioning the web treatment head 400 and the web treatment tool 450 relative to each other and to the web material so as to allow them to interact each with one of the opposite surfaces of the web material(s);
(f) treating the first and the second web regions jointly by cooperatively operating the web treatment head 400 and the web treatment tool 450 of the treatment unit, thereby forming the structure of the article;
(g) removing the web treatment head from the gap and/or the article from the head;
(h) optionally further treating the article;
(i) removing the article from the web handling equipment.

The web materials may be made of films, textile, woven, or non-woven webs, or composites thereof. The first and second regions may belong to different webs; which may be of the same or different material type, orientation, or which may have undergone different pre-treatments. The webs material(s) may be pre-shaped webs, optionally forming a closed structure like a belt or a hoop. The method may further include the use of other materials, optionally preassembled pieces, bulk material, or fluids, and several process steps may be executed on one piece of web material. A preferred treatment step is the connecting of two regions, but further treatment steps may be included, such as cutting, pressing, or mechanically activating of webs.

The present invention applies to a variety of articles. Without intending any limitation to the application, the particular executions and resulting advantages as provided for particular disposable absorbent articles are now explained in more detail.

Feminine care pads are typically used by being inserted into the pants or panty of a wearer, covering the perineal region. They might be designed as menstrual pad, as incontinence pad, or as panty liner.

The design differences will then vary with regard to size and absorbency of the pad. Thus, panty liners will typically have a small absorbent capacity, but it is important that they have little or no occlusive effect, which can be further enhanced by using air permeable ("breathable") materials. The present invention further allows for a non-contact zone between the pad and the body of the wearer.

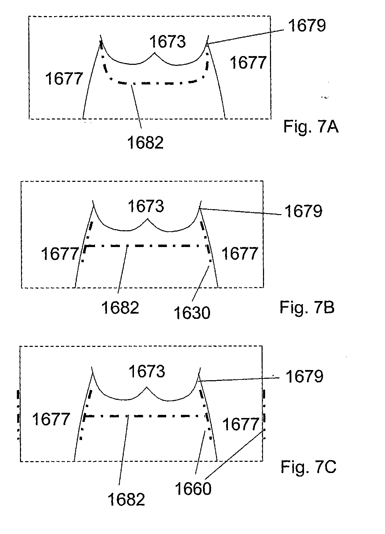

Fig. 7 shows "fit lines" 1682, which are a schematic cross-sectional view of the article in an in-use configuration and positioning in the crotch region of the wearer, here indicated by legs 1677, lower torso 1673, and crotch crease 1679 between these. Fig. 7 A, B, and C show the "fit line" of the above described cup-like shaped article as in Fig. 1D, 2B, and 2A, respectively, and the products according to the present invention comprising a hoop as shown in Fig 7G and 2H respectively, provide improved leakage prevention, sustained dynamic fit and comfort, and in particular by providing a non-contact zone between the article and the skin

an article according to Fig 1D still relies on a further support means, and primarily relies on the anatomically formed cup shape. Whilst also an article according to Fig 2B requires a further support means, the seal cuff provides a significantly improved leakage protection. First, it provides a sealing band on the inner thigh region. Second, this seal cuff extends upwardly from the pad such that leakage is further prevented. Such as design is depicted and described hereinabove in connection with Fig. 2B. The second execution with a leg encircling hoop (as shown in Fig. 2A) may be used practically independently from a further support means, as the pad may be held in place by the leg hoops and may be held in shape by the structural connection between the leg hoops and the centre crotch regions.

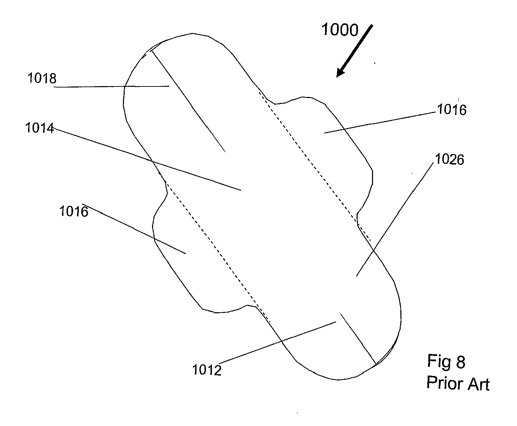

For articles intended for higher loading, it may be highly desirable to have leakage protection at the side portions of the pad, to prevent soiling or staining of the outer garment, if it is loaded beyond the absorbency capacity or acquisition capability (gush handling) of the pad. Conventional pad designs typically attempt to address this by providing the article with side crotch regions ("wings"), which are folded downwardly around the leg cuff of the pants. This, however, requires a 180° fold, which is typically achieved by attaching the wings e.g. by adhesive patches to the outer side of the panty, i.e. on the opposite side of the pad (see Fig. 8) If it were not folded by 180°, the folding would support an upward bulging of the centre crotch region.

Pant type articles may be used as single use non-absorbent garments, e.g. when being worn in operation theatres. Such non-absorbent articles may also be useful for multiple use, optionally including washability, e.g. when being used as a menstrual pant or as a support pant for incontinence pads in combination with an absorbent pad. The pant structure of the articles may be permanently closed, or openable, e.g. at the waist to ease donning of the article.

Conventional baby or adult incontinence diapers typically comprise a portion to fit in the crotch region of the wearer (similar to pads) extending fore- and rearwardly upwards towards the waist and the back of the wearer, which further comprise closeable means to attach the diaper around the waist or hips of the wearer.

In contrast to such conventional diapers, the present invention allows distinct advantages, in particular with regard to leakage sealing, comfort and continued dynamic fit.

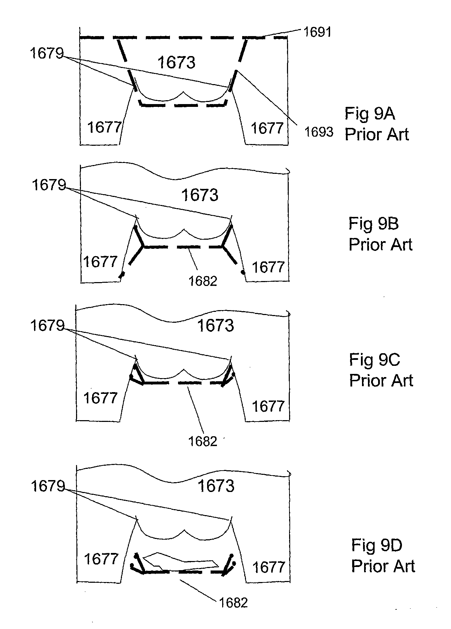

As can be seen in Fig. 9A, conventional diapers rely on a strong anchorage around the waist or hips of the wearer, as indicated by a waist force line 1691 showing a schematic cross-section view of a crotch region. The leakage sealing towards the legs is typically achieved by elastic elements, which are affixed close to the waist regions, and run though the crotch crease (i.e. the crease 1679 formed by a leg 1677 and the lower torso 1673). This creates a force line as indicated by the waist-crotch-crease force line 1693. Due to this force line, all elastic elements typically fit into this crotch crease, regardless where they were positioned upon donning of the article. In Fig. 9B, an "initial" fit line 1682 is indicating the intended positioning of the article, but after a certain wear time and movement, the "in-use fit line" will look as indicated in Fig. 9C when the article is not loaded, or as indicated in Fig. 9D when the article is loaded.

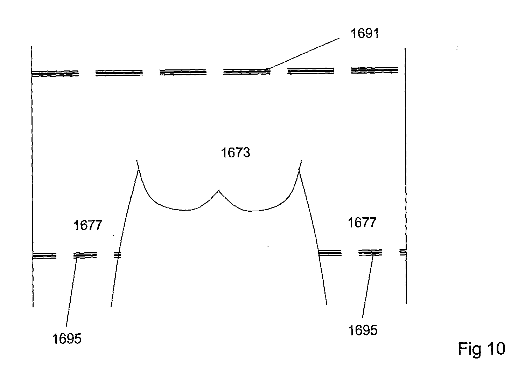

The present invention, however, allows to place a leg encircling hoop which will - in addition to forming the cup-like shape - very advantageously improve the fit. As can be seen in Fig. 10, the force lines to hold the pant around the waist 1691 and to seal at the legs 1695 are decoupled. Due to a leg seal cuff lying against the leg over its full width, very gentle and comfortable sealing may be achieved, which will also withstand dynamic movement of the wearer.

As described with Fig. 3, a pant design according to the present invention may comprise a piece comprising the front and rear regions as well as the centre crotch region, two side panel pieces and leg seal cuff pieces.

For example, the leg seal cuff pieces may be elasticised, and the larger side panel pieces may be made of cheaper not-elastic webs. Alternatively, the side panel pieces may be elasticised, and optionally have a varying degree of elastication, e.g. in the waist region as compared to the leg region or to the hip region.

A further design of a pant type article according to the present invention is shown if Fig. 4, as discussed in the above. In this case, there is a leg hoop as a side crotch region, encircling the full leg, which is connected to the centre crotch region as well as to the front and rear regions, which extend more laterally outwardly than in the design as depicted in Fig. 3, such that these can be folded and optionally connected appropriately so as to form the complete hoop structures both at the waist as well as at the legs.

A particularly useful application of the present invention relates to designs, such as for diapers, comprising a so called secondary topsheet.

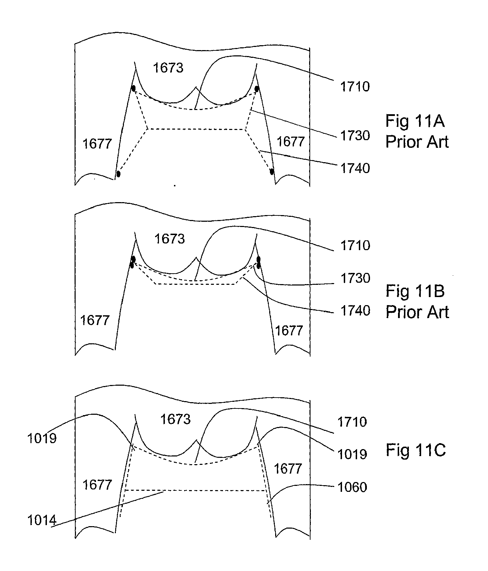

Secondary topsheets are known in conventional diapers, such as described in US-B-4990147, or WO-A-04/04331, and refers to a design, where in addition to a first topsheet covering the absorbent core, an additional web material is applied overlaying the first topsheet (i.e. being oriented towards the wearer). During use, this secondary topsheet is positioned close to or even in contact with the skin of the wearer, in particular in the rear buttocks regions. Optionally, and often preferably, the secondary topsheet may be at least partially elasticized. It further comprises an opening, which is positioned in registry with the anal opening of the wearer, such that faeces may penetrate through this opening into a void space between the first and the secondary topsheet. Because of the barrier properties of the secondary topsheet for faeces, the skin of the wearer is there protected from the often aggressive faecal material. As these barrier properties may also hinder urine to pass through the secondary topsheet, a further opening may be provided in the urine loading region, or the urine loading region of the secondary topsheet may be specifically treated so as to allow urine to readily penetrate through. This may be achieved by a hydrophilization treatment, which may be executed as being permanent, i.e. not being washed away by urine penetrating through. Alternatively, the secondary top sheet may end at the point of urine exit such that the primary top sheet forms the layer next to the skin in this particular area. Fig 11 A shows a conventional secondary topsheet design with a secondary topsheet 1710 being initially positioned stretched between the barrier cuff elastics 1730 whilst the leg elastics 1740 keep the void space open. However, as discussed in the above, in such conventional diaper designs the leg elastics tend to move upwardly towards the crotch crease 1679 (Fig. 11B), thereby closing the void space and rendering the secondary topsheet non-functional.

In contrast, the present invention provides a particularly well functioning secondary topsheet design. As depicted in Fig. 11C, the secondary topsheet 1710 is attached to the upper perimeter 1019 of the leg seal cuff 1060. As the leg seal cuff will stay on the upper thigh also during movement, the void space of the cup-shaped crotch region will be maintained whilst the apertured secondary topsheet 1710 will stay in close contact with the skin of the wearer. Thus, faeces penetrating through the opening of the secondary topsheet, which is positioned in registry with the anal opening of the wearer, may be accommodated in this void space, and yet be separated from the skin of the wearer. A particular benefit of this design lies in the fact that only minor cross-directional forces are required to keep the opening of the secondary top sheet in the desired position and open. There are also no significant forces acting in longitudinal direction of the product required to keep the secondary top sheet against the skin of the wearer, like in conventionally designed articles, which try to lift the secondary top sheet against the skin primarily by means of forces in longitudinal direction, thus pulling the products down.

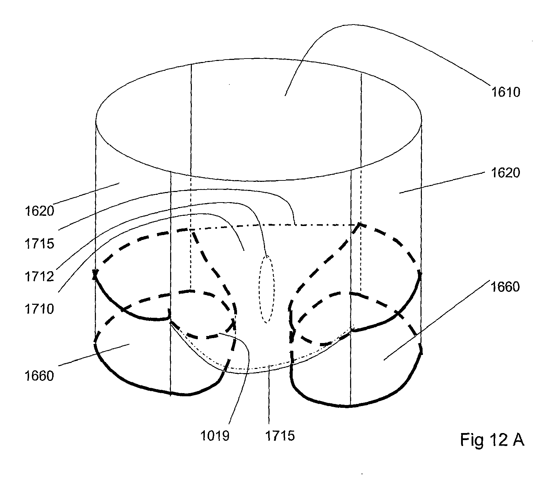

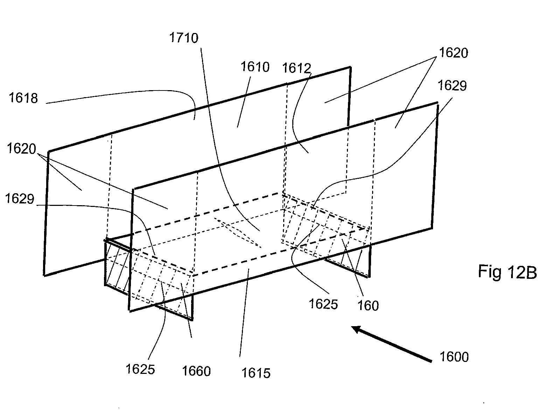

In a first execution of such a product, see Fig. 12 A and B, the leg hoops 1660 are attached to the backsheet side of the article, i.e. away from the wearer, as also depicted in Fig. 4 A and B. The centre crotch region, which may comprise an absorbent core including the first topsheet may be cup-like shaped as described hereinabove by being connected to a leg hoop 1660. The secondary topsheet 1710 comprising an aperture 1712 in registry with the anal opening of a wearer is connected to the front and rear regions along bond lines 1715 and maybe connected to the upper edge 1019 of the leg hoop and thus be stretched close to the skin of the wearer. Because of radial fit forces of the leg hoop around the leg, it remains in the configuration as depicted in Fig. 11C also during use, withstanding the dynamic stress and thusly keeping the void space open.

The production of such a product follows essentially the principles as laid out hereinabove by attaching a leg element (i.e. the leg seal cuff 1630 or leg hoop 1660) to the outer surface of the article (i.e. oriented away from the wearer when in an in-use configuration). The secondary topsheet is connected to the front and rear region, such as by gluing or thermal bonding and it may be connected to the upper perimeter of the leg elements, e.g. after these have been unfolded into the in-use configuration. In this design, it might be preferable to not permanently bond the secondary topsheet to the leg seal cuff or leg hoop, but to design its width such that it extends sideward into the leg elements, and thus may be held by the friction force generated by the contraction of the leg elements. Alternatively, the secondary TS may be permanently bonded at least partially along its side lines to the inner surfaces of the leg hoops or leg cuffs, which may be temporarily backfolded prior to addition of the secondary topsheet further upstream in the process, such that the upper edge of the inner part of the leg hoop or leg cuff is essentially in a similar position as during use.

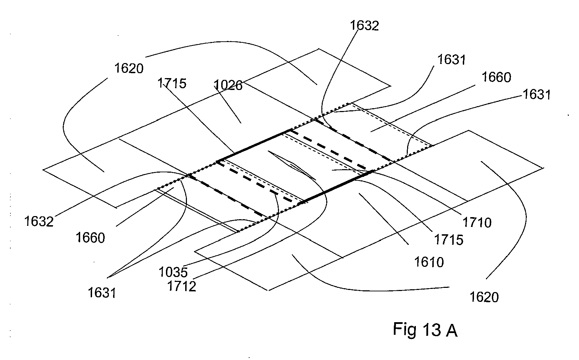

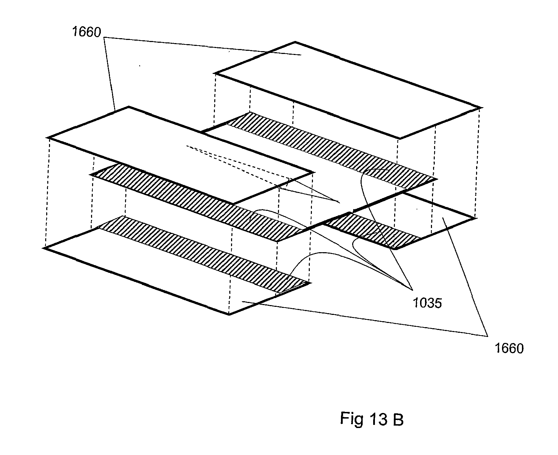

A second execution of a pant product comprising a secondary topsheet (see Fig. 13) comprises leg elements, which are attached to the upper, wearer oriented side of the article, e.g. on a primary topsheet overlaying the absorbent core. In contrast to the previous design, however, the lateral outward side portions of the leg elements will not be folded 90° upwards for creating the cup-like shape, but rather downwardly. Since the lateral inward side portions of the leg elements are closer to the article centreline than the line connecting leg elements and side crotch areas (line 1632 in Fig 13A), the inner parts of the leg elements are folded upwardly at the time the outward portions are folded downwards.

Thus, the basic structure (prior to application of the leg elements and the 2^{nd} topsheet) corresponds essentially to the one as shown in Fig. 13 with wider front and rear regions adapted to encircle the leg hoop. Therein, as shown in Fig. 13 A with a partly exploded and spread out view of the webs forming such an article, a leg hoop 1660 is attached on the upper, i.e. wearer oriented side 1026 via bonding line 1632.

The secondary topsheet 1710 is positioned between the outer and inner part of leg hoop 1660, and connected via the hoop connecting line or region 1035 to the inner layer thereof (as shown in an exploded partial view of these features - Fig. 13B). This region is shown as having a straight line perimeter as to not negatively affect the forming of the cup-like shape upon folding of the leg hoop into an upstanding position.

In this execution, because of the lack of a curved connection / fold line between the leg hoop and the centre crotch region, the side portions of the centre crotch region preferably are more supple to allow better adaptation to the body contours.

## Claims

1. An article comprising a cup-like shaped structure,
said cup-like shaped structure being adapted for being concavely positioned in the crotch of a wearer during use,
said article comprising
a front and/or a rear region,
and a crotch region;
said crotch region comprising
a centre crotch region,
and two side crotch regions laterally outwardly of said centre crotch region forming a hoop which is essentially non-unitary with said centre crotch region,
each of these regions having a first surface, oriented towards the wearer during use and an opposite surface, whereby said opposite surfaces of said side crotch regions being connected along connecting lines to said opposite surface of said center crotch region, whereby the connecting line is curve-linearly shaped said cup like structure being formed and maintained during use by connecting said side crotch regions along connecting lines to said centre crotch region, whereby at least one of the connecting lines or optional fold-lines is curve-linearly shaped.

2. An article according to claim 1,
wherein said side crotch regions are forming a hoop, said hoop having preferably a width of more than 1 mm, more preferably of more than 5 mm, and even more preferably of more than 10 mm, but preferably of less than 100 mm, more preferably of less than 70 mm, and even more preferably of less than 40 mm.

3. An article according to any of the preceding claims,
comprising a waist hoop connected to or formed by said front and rear regions.

4. An article according to any of the preceding claims,
further comprising side panel materials connected to said front and rear regions and to said side crotch regions, said side panel materials optionally being non-unitary materials.

5. An article according to any of the preceding claims,
wherein said article is a disposable absorbent article, a disposable non-absorbent article, a baby diaper, a feminine hygiene product, an adult incontinence product, a piece of single or limited use underwear, a piece of operation theatre garment, or a tight fitting elasticised garment.

6. An article according to any of the preceding claims,
wherein said front, rear, centre, or side regions comprise web materials, preferably non-wovens, films, or composites thereof

7. An article according to any of the preceding claims,
wherein said front, rear, centre, or side regions comprise at least partially elasticized materials.

8. An article according to any of the preceding claims,
wherein said regions exhibit a cantilever stiffness of less than 20, preferably less than 20, or more than 1.5, preferably 2, when tested according to ASTM-D-1388, optionally exhibiting directionally non-uniform stiffness.

9. An article according to any of the preceding claims,
wherein said connecting lines are continuous or intermittently curve-linear, single or multiple lines, intercepting lines, or defined as the perimeter of a connecting region.

10. An article according to any of the preceding claims,
wherein said connecting is permanent, preferably a non-sewn connection, optionally with tear open aids, or non-permanent of the hook-loop system type, or a refastenable adhesive system.

11. An article according to any of the preceding claims,
further comprising any or all of the following elements:
(i) an absorbent core;
(ii) a barrier leg cuff;
(iii) a secondary topsheet;
(iv) lotion application to body facing surfaces.

## Patentansprüche

1. Ein Artikel, der eine becherartig geformte Struktur beinhaltet,
wobei die becherartig geformte Struktur eingerichtet ist, um während des Gebrauchs im Schrittbereich eines Benutzers konkav positioniert zu werden,
wobei der Artikel einen vorderen und/oder einen hinteren Bereich sowie einen Schrittbereich beinhaltet,
wobei der Schrittbereich einen mittleren Schrittbereich und zwei Seitenschrittbereiche lateral außerhalb des mittleren Schrittbereichs, die Beinbündchen formen, die im Wesentlichen nicht einheitlich mit dem mittleren Schrittbereich sind, beinhaltet,
wobei jeder dieser Bereiche eine erste Oberfläche aufweist, die während des Gebrauchs zu einem Benutzer hin ausgerichtet ist, und eine zweite gegenüberliegende Oberfläche, wobei die gegenüberliegenden Oberflächen der Seitenschrittbereiche mit der entsprechenden gegenüberliegenden Oberfläche des mittleren Schrittbereichs durch Verbundlinien verbunden sind,
wobei diese Verbundlinien kurvenförmig ausgebildet sind,
wobei die becherartig geformte Struktur geformt und während des Gebrauchs dadurch aufrechterhalten wird, dass die Seitenschrittbereiche durch Verbindungslinien zu dem mittleren Schrittbereich verbunden sind, wobei zumindest eine der Verbindungslinien oder optionalen Faltlinien kurvenförmig ausgebildet ist.

2. Ein Artikel gemäß Anspruch 1,
wobei die Seitenschrittbereiche Bündchen bilden, vorzugsweise mit einer Breite von mehr als 1 mm, noch bevorzugter von mehr als 5 mm, und noch mehr bevorzugt von mehr als 10 mm, aber bevorzugterweise weniger als 100 mm, noch bevorzugterweise weniger als 70 mm und noch mehr bevorzugterweise weniger als 40 mm.

3. Ein Artikel gemäß einem der vorhergehenden Ansprüche,
der außerdem ein Bauchbündchen beinhaltet, welches mit einem vorderen und hinteren Bereich verbunden ist oder aus diesen geformt wird.

4. Ein Artikel gemäß einem der vorhergehenden Ansprüche,
der außerdem Seitenteile enthält, die mit den vorderen und hinteren Bereichen sowie den Seitenschrittbereichen verbunden sind,
wobei die Seitenteile optional nicht-einheitliche Materialien sein können.

5. Ein Artikel gemäß einem der vorhergehenden Ansprüche,
wobei der Artikel ein absorbierender Wegwerfartikel, ein nicht absorbierender Wegwerfartikel, eine Baby-Windel, ein Damenhygieneprodukt, ein Inkontinenzprodukt für Erwachsene, einmal oder mehrfach nutzbare Unterwäsche, ein Stück Bekleidung zur Benutzung während Operationen oder ein eng sitzendes elastisches Bekleidungsstück ist.

6. Ein Artikel gemäß einem der vorhergehenden Ansprüche,
wobei der vordere, der hintere, der mittlere oder die Seitenbereiche Bahnmaterialien, vorzugsweise Vliese, Folien oder Kombinationen dieser, beinhalten.

7. Ein Artikel gemäß einem der vorhergehenden Ansprüche,
wobei der vordere, der hintere, der mittlere oder die Seitenbereiche zumindest teilweise elastische Materialien beinhalten.

8. Ein Artikel gemäß einem der vorhergehenden Ansprüche,
wobei die Bereiche eine Kragsteifheit von weniger als 20 oder mehr als 1,5, vorzugsweise mehr als 2 aufweisen, wenn sie gemäß ASTM-D-1388 vermessen werden, wobei optional die Kragsteifheit in verschiedenen Richtung unterschiedlich sein kann.

9. Ein Artikel gemäß einem der vorhergehenden Ansprüche,
wobei die Verbindungslinien durchgehende oder unterbrochene Kurvenlinien sind, einzelne oder Mehrfachlinien, sich schneidende Linien oder durch den Umriss eines Verbindungsbereiches definiert werden.

10. Ein Artikel gemäß einem der vorhergehenden Ansprüche,
wobei die Verbindung dauerhaft, vorzugsweise nicht genäht, wahlweise mit Aufreißhilfen, oder nicht-dauerhaft als Haken-Schlaufen-Verbindung oder durch ein System mit wiederverschließbaren Leimen ist.

11. Ein Artikel gemäß einem der vorhergehenden Ansprüche,
der eins oder mehrere der folgenden Elemente beinhaltet:
(i) ein absorbierendes Saugkissen;
(ii) eine Beindichtmanschette;
(iii) ein zweites Abdeckvlies;
(iv) ein Lotion, die auf körperzugewandten Oberflächen aufgebracht ist.

## Revendications

1. Un article comprenant une structure comme une coupelle,
cette structure comme une coupelle étant adaptée pour être positionnée concavement dans la région entrejambes d'un usager pendant l'utilisation,
cet article comprenant
une région avant et/ou une région derrière et une région entrejambes, ladite région entrejambes comprenant
une région entrejambes centrale,
et deux régions latérales de cette région entrejambes au dehors de cette région entrejambes centrale, lesdites régions latérales de cette région entrejambes formant un col qui est essentiellement non-unitaire avec ladite région entrejambes centrale, dont tous ces régions ont une première surface qui est dirigée vers un usager pendant l'utilisation, et une surface opposée,
tel que lesdites surfaces opposées de les régions latérales de cette région entrejambes sont reliée par des lignes de connexion, qui suivent une trajectoire courbée, avec la surface opposée de la région entrejambes centrale,
tel que cette structure comme une coupelle est formée et maintenue pendant l'utilisation par la connexion de les régions latérales de la région entrejambes par des lignes de connexion à la région entrejambes centrale ce que des lignes de connexion ou optionnellement de pliage suivent une trajectoire courbée.

2. Un article selon la revendication 1,
dans lequel lesdites régions latérales de cette région entrejambes forment un col ayant une largeur de plus de 1 mm, préférablement plus de 5 mm, plus préférablement plus de 10 mm, mais préférablement moins de 100 mm, plus préférablement moins de 70 mm, encore plus préférablement moins de 40 mm.

3. Un article selon l'une quelconque des revendications précédentes comprenant un col de la taille relié à ou formé par la région avant et la région derrière.

4. Un article selon l'une quelconque des revendications précédentes comprenant en outre des panneaux latéraux qui sont reliés à lesdites régions avant et derrière et aux régions latérales de cette région entrejambes, tandis que ces panneaux latéraux optionnellement peuvent être des matériaux non-unitaires.

5. Un article selon l'une quelconque des revendications précédentes,
cet article étant un article absorbant jetable, un article non-absorbant jetable, une couche bébé, un produit pour la hygiène féminine, un produit pour l'incontinence adulte, un sous-vêtement pour l'utilisation unique ou multiple, un vêtement pour une salle d'opération, ou un vêtement moulant élastique.

6. Un article selon l'une quelconque des revendications précédentes,
dans lequel les régions avant, arrière, centrale, ou latéral comprennent une bande de matériaux, préférablement un non-tissé, un film ou une combinaison.

7. Un article selon l'une quelconque des revendications précédentes,
dans lequel les régions avant, derrière, centrale, ou latéral comprennent des matériaux qui sont au moins partiellement élastique.

8. Un article selon l'une quelconque des revendications précédentes,
dans lequel les régions comportent une rigidité en porte-à-faux de moins de 20 ou plus de 1,5, préférablement plus de 2, déterminée par la méthode ASTM-D-1388, dont la valeur peut optionnellement varier selon la direction.

9. Un article selon l'une quelconque des revendications précédentes,
dans lequel les lignes de connexion sont continuellement ou discontinuellement courbées, une seul ligne ou des lignes multiple, optionnellement cisaillée(s), ou peuvent être formées par le périmètre d'une région de connexion.

10. Un article selon l'une quelconque des revendications précédentes,
dans lequel la connexion est permanente, préférablement non-cousue, optionnellement avec une aide pour l'ouvrir, ou non-permanente de la type crochet - boucles ou d'un système des adhésives refermable.

11. Un article selon l'une quelconque des revendications précédentes comprenant un ou plusieurs des éléments suivant(s) :
(i) un noyau absorbant ;
(ii) un revers de jambe de barrière;
(iii) une couche de feuille supérieure secondaire;
(iv) une application de lotion au surfaces qui sont dirigée vers l'usager.
